Europäisches Patentamt

**European Patent Office**

**Office européen des brevets**

(19)

(11) Veröffentlichungsnummer: **0 053 245**
**B1**

(12)                    EUROPÄISCHE PATENTSCHRIFT

(45) Veröffentlichungstag der Patentschrift:
**30.11.83**

(51) Int. Cl.³: **C 07 C 47/09**

(21) Anmeldenummer: **81108200.7**

(22) Anmeldetag: **12.10.81**

(54) Verfahren zur Abtrennung organischer Jod-Verbindungen von Acetaldehyd.

(30) Priorität: **29.11.80 DE 3045105**

(43) Veröffentlichungstag der Anmeldung:
**09.06.82 Patentblatt 82/23**

(45) Bekanntmachung des Hinweises auf die Patenterteilung:
**30.11.83 Patentblatt 83/48**

(84) Benannte Vertragsstaaten:
**BE DE FR GB IT NL**

(56) Entgegenhaltungen:
**keine**

(73) Patentinhaber: **BASF Aktiengesellschaft,
Carl-Bosch-Strasse 38, D-6700 Ludwigshafen (DE)**

(72) Erfinder: **Hartmann, Horst, Lindenstrasse 45,
D-6737 Boehl-Iggelheim (DE)**
Erfinder: **Hochstein, Waldhelm, Dr., Am Wurmberg 4,
D-6713 Freinsheim (DE)**
Erfinder: **Kaibel, Gerd, Robert-Bosch-Strasse 4,
D-6840 Lampertheim (DE)**
Erfinder: **Mueller, Franz-Josef, Dr.,
Mueller-Thurgau-Weg 1, D-6706 Wachenheim (DE)**

Verfahren zur Abtrennung organischer Jod-Verbindungen von Acetaldehyd

Die vorliegende Erfindung betrifft ein neues Verfahren zur Abtrennung organischer Jod-Verbindungen, wie vor allem Methyljodid, von Acetaldehyd.

Mit organischen Jodverbindungen, vor allem mit Methyljodid verunreinigter Acetaldehyd fällt bei der sogenannten Homologisierung von Methanol mittels eines Katalysatorsystems aus Carbonylkomplexen von Metallen der VIII. Gruppe des Periodensystems und Jodverbindungen als Aktivatoren an. Näheres zu dieser Reaktion, auf deren Einzelheiten es im Rahmen der vorliegenden Erfindung nicht ankommt, ist der Monographie von Falbe, »Chemierohstoffe aus Kohle«, Georg-Thieme-Verlag, 1977, Seite 329 ff sowie den dort zitierten Originalarbeiten zu entnehmen.

Verwendet man Jodide als Aktivatoren, so erhält man nach Abtrennung der Katalysatormetalle stets einen rohen Acetaldehyd, der mit geringen Mengen Methyljodid sowie sonstigen organischen Jodverbindungen, die sich bei der Homologisierungsreaktion bilden, verunreinigt ist.

In aller Regel bereitet die meist unumgängliche weitgehende Entfernung von jodorganischen Verunreinigungen aus organischen Verbindungen erhebliche Schwierigkeiten. Im vorliegenden Falle kommt hinzu, daß allgemein bekannte chemische Methoden wie Alkalibehandlung, Reduktion oder Oxidation von vornherein ausscheiden, will man nicht beträchtliche Verluste des gegen derartige Reaktionen höchst empfindlichen Acetaldehydes in Kauf nehmen. Eine Fraktionierung hingegen ist nicht möglich, weil Acetaldehyd und Methyljodid praktisch nicht voneinander zu trennen sind.

Auch das in der DE-OS 2 940 751 empfohlene Verfahren zur Abtrennung organischer Jodverbindungen von Carbonylierungsprodukten des Methylacetats, nach welchem das Jod der organischen Jodverbindungen bei höherer Temperatur mittels Alkalimetallacetaten in die nichtflüchtigen Alkalimetalljodide überführt wird, ist nicht nur wegen seiner verfahrenstechnischen Umständlichkeit unbefriedigend, sondern läßt sich auch wegen der Alkaliempfindlichkeit des Acetaldehyds nicht mit Erfolg auf dessen Reinigung anwenden.

Der Erfindung lag daher die Aufgabe zugrunde, Acetaldehyd auf einfachere und wirkungsvollere Weise von organischen Jodverbindungen zu befreien als bisher.

Demgemäß wurde gefunden, daß sich organische Jodverbindungen auf elegante Weise von Acetaldehyd abtrennen lassen, wenn man den Acetaldehyd einer Azeotropdestillation mit einem Kohlenwasserstoff, der unter Normaldruck bei 25—55°C siedet, unterwirft.

Geeignete Kohlenwasserstoffe sind n-Pentan, Isopentan (Methylbutan), Cyclopentan und 2,2-Dimethylbutan.

Da sich das Isopentan für den erfindungsgemäßen Zweck besonders gut eignet, sei die Erfindung im folgenden für diesen Fall näher erläutert. Für die Verwendung der übrigen definitionsgemäßen Kohlenwasserstoffe gilt bis auf den Unterschied etwas abweichender Zusammensetzungen der azeotropen Gemische sowie etwas abweichender Destillationstemperaturen das gleiche.

Da überraschenderweise festgestellt wurde, daß das Acetaldehyd/Isopentan-Azeotrop nur noch äußerst geringe Mengen an Jodverbindungen enthält, kommt es auf deren Anfangskonzentration im rohen Acetaldehyd nicht an.

Handelt es sich um den technisch wichtigsten Fall, nämlich die Abtrennung des Methyljodids und geringer Mengen sonstiger jodorganischen Verbindungen, die man aus praktischen Gründen als Methyljodid rechnet, so setzt sich das unter Normaldruck bei 11°C siedende Azeotrop aus rund 25 Gew.-% Acetaldehyd und rund 75 Gew.-% Isopentan zusammen. Diese Zusammensetzung ändert sich mit steigendem Druck bis zu etwa 4 bar praktisch nicht. In allen Fällen liegt die Methyljodid-Konzentration im Destillat unter 0,5 ppm.

Wegen der destillationstechnisch leichteren Kühlung bevorzugt man in der Praxis einen Druck von 2 bar (Siedepunkt des Azeotrops 29°C) bis 3 bar (Siedepunkt 42°C).

Zur Gewinnung des reinen Acetaldehyds kann man das Acetaldehyd/Isopentan-Azeotrop zunächst kondensieren und den Acetaldehyd hieraus danach in an sich bekannter Weise mit Wasser extrahieren, jedoch ist es auch möglich, das Azeotrop unmittelbar aus der Gasphase heraus in Wasser aufzunehmen. In beiden Fällen sind hierzu pro Kilogramm Acetaldehyd je nach dem tolerierbaren Gehalt des Kohlenwasserstoffes im Acetaldehyd etwa 1—5 kg Wasser erforderlich.

Der Acetaldehyd läßt sich dann wie üblich destillativ aus der wäßrigen Phase gewinnen.

Man kann die erfindungsgemäße Azeotrop-Destillation in Kolonnen beliebiger Bauart ausführen, deren theoretische Bodenzahl vorzugsweise zwischen 20 und 35 beträgt.

Eine besonders vorteilhafte Ausgestaltung des erfindungsgemäßen Verfahrens besteht in dessen Integrierung in das Verfahren zur Gewinnung von Acetaldehyd aus Reaktionsgemischen der Homologisierung von Methanol gemäß der DE-A-3 019 765.

Dieses Verfahren zur Aufarbeitung der Homologisierungsgemische auf Acetaldehyd, die neben Acetaldehyd im wesentlichen Acetaldehyddimethylacetal, Methanol, Methylacetat und Wasser enthalten, ist dadurch gekennzeichnet, daß man

a) das Homologisierungsgemisch bei einer Temperatur in den mittleren Teil einer Kolonne I gibt, bei welcher das Methylacetat oder sein azeotropes Gemisch mit Methanol, nicht aber das Methanol gänzlich verdampft,

b₁) im Gegenstrom zu den aufsteigenden Dämpfen flüssige wäßrige Säure leitet, die im oberen Viertel der Kolonne I zugeführt wird und welche eine Spaltung des Acetaldehyddimethylacetals in Acetaldehyd und Methanol bewirkt, oder

b₂) anstelle der wäßrigen Säure lediglich Wasser verwendet und die Acetspaltung mittels eines festen sauren Ionenaustauschers bewirkt,

c) vom Kopf der Kolonne I ein dampfförmiges Gemisch aus Methylacetat und Acetaldehyd abzieht, welches in einer Kolonne II in seine Komponenten zerlegt wird,

d) im unteren Viertel der Kolonne I ein flüssiges Gemisch aus Methanol und Wasser abzieht, welches in einer Kolonne III in seine Komponenten zerlegt wird, und wenn man

e) einen Teil der im Sumpf der Kolonne I anfallenden wäßrigen Säure (Variante b₁) bzw. des Wassers (Variante b₂), zusammen mit untergeordneten Mengen sonstiger Produkte aus dem System entfernt und den restlichen Teil dieser wäßrigen Säure bzw. des Wassers mit dem im Sumpf der Kolonne III anfallenden Wasser vereinigt und die vereinigte wäßrige Lösung nach Zugabe einer Säure wieder in das obere Viertel der Kolonne I zurückführt.

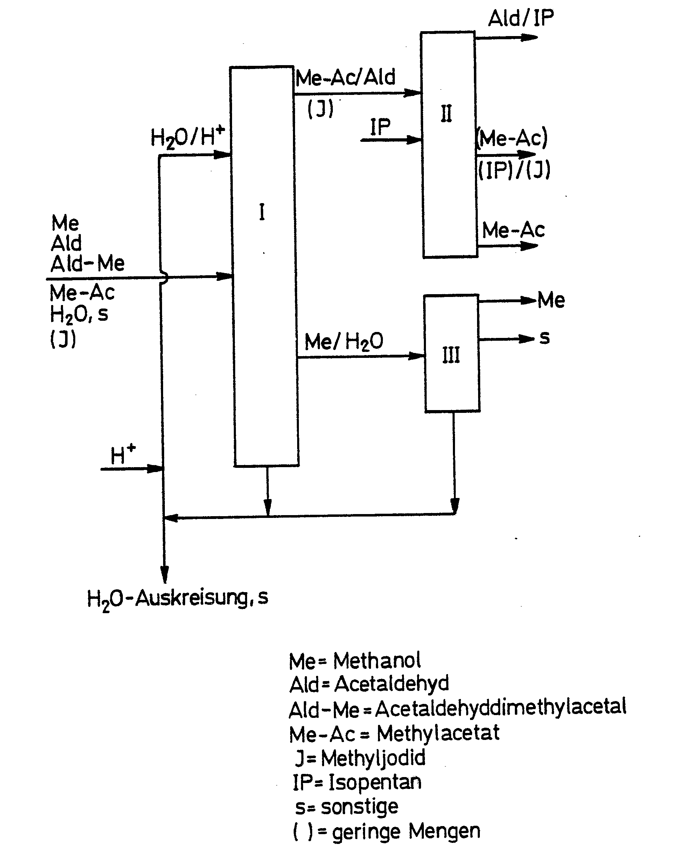

Nimmt man im Verfahrensschritt (c) die Trennung von Methylacetat und Acetaldehyd erfindungsgemäß in Gegenwart des Isopentans vor, so erhält man aus einer einzigen integrierten Anlage zur Aufarbeitung von katalysatorfreien Reaktionsgemischen aus der Methanol-Homologisierung unmittelbar jodfreien Acetaldehyd, sofern die Homologisierung, wie allgemein üblich, mit Jodverbindungen als Aktivatoren vorgenommen wurde. Die Zeichnung veranschaulicht diese Verfahrensweise.

In einer Variante dieses integrierten Verfahrens kann man die Gewinnung des Acetaldehyd/Isopentanazeotropes auch in den Oberteil der Kolonne I verlegen und das Methylacetat in einer Abtriebsseitenkolonne abtrennen.

In beiden Fällen gelangt das Methyljodid in das Methylacetat. Dieses Gemisch kann destillativ in der gleichen Apparatur in eine jodfreie und, als Seitenabzug, in eine jodhaltige und Isopentan enthaltende Fraktion zerlegt werden. Die jodhaltige Fraktion kann dann in die Homologisierungsstufe zurückgeführt werden. Hierdurch wird, was verfahrenstechnisch besonders vorteilhaft ist, ein geschlossener Jodkreislauf ohne irgendwelche Umwandlungen der Jodverbindungen ermöglicht.

Das erfindungsgemäße Verfahren beruht auf der Erkenntnis, daß Acetaldehyd durch azeotrope Destillation mit den definitionsgemäßen Kohlenwasserstoffen von Methyljodid sowie sonstigen organischen Jodverbindungen befreit werden kann, unabhängig davon, wie die Jodverbindungen in den Acetaldehyd gelangt sind.

Große praktische Bedeutung hat dieses Trennproblem bisher jedoch nur bei der Homologisierung von Methanol, soweit man diese Reaktion durch Wahl relativ geringer Umsätze und Vermeidung eines zu großen Wasserstoffpartialdruckes auf der Stufe des Acetaldehyds abbricht. Dieser Partialhomologisierung ist meist der Vorzug vor der vollständigen Homologisierung zum Ethanol zu geben, denn der Acetaldehyd ist vielseitiger verwendbar als Ethanol.

Allgemein nimmt man die Partialhomologisierung des Methanols bei 150 bis 200°C, einem CO-Partialdruck von 100—200 bar und einem $H_2$-Partialdruck von 100—200 bar, vorzugsweise in Gegenwart von Cobalt- oder Rhodiumcarbonylkomplexen vor, in denen ein Teil des Carbonyls auch durch andere Liganden wie Trialkylphosphine und -phosphite oder Triphenylphosphin ersetzt sein kann. Die Menge dieser Katalysatoren, auf das Metall berechnet, beträgt etwa 0,01—0,1 Mol-% des Methanols. Durch Jodverbindungen wie Jodwasserstoff, Alkalijodide oder insbesondere Methyljodid, welches sich unabhängig von der Jodquelle stets bildet, wird die Homologisierungsreaktion stark begünstigt. Man verwendet die Jodverbindungen etwa in der 1,5- bis 3fachen molaren Menge des Katalysatormetalls, so daß der Reaktionsaustrag neben 30 bis 75 Gew.-% Methanol, 2 bis 10 Gew.-% Acetaldehyd, 8 bis 30 Gew.-% Acetaldehyddimethylacetal, 5 bis 10 Gew.-% Methylacetat und 10 bis 20 Gew.-% Wasser etwa 0,01 bis 0,1 Gew.-% Methyljodid enthält.

Verwendet man nicht-flüchtige Katalysatoren (z. B. Rhodium/Triphenylphosphin/Carbonyl-Komplexe), so kann das Reaktionsgemisch gasförmig abgetrennt und den weiteren Aufarbeitungsschritten zugeführt werden. Im Falle flüchtiger Metallcarbonyle, z. B. Dicobaltoctacarbonyl, ist zuvor eine Zersetzung des Katalysators in die entsprechenden Metallsalze, z. B. Cobaltacetat, erforderlich, die man z. B. mit Luft vornehmen kann.

Arbeitet man das katalysatorfreie Reaktionsgemisch zunächst durch Grobfraktionierung auf den rohen Acetaldehyd auf, so enthält dieser etwa 100—500 ppm Methyljodid. Wenn man hieran eine diskontinuierliche Reinigung nach dem erfindungsgemäßen Verfahren anschließt, so empfiehlt es sich bei Verwendung einer Kolonne mit der an sich ausreichenden Anzahl von 15 bis 30 theoretischen Böden, die Methyljodid-Konzentration im Sumpf der Destillationskolonne nicht über 1—2 Gew.-% ansteigen zu lassen, damit der Partialdruck des Methyljodids nicht zu hoch wird und die Trennleistung einer solchen Kolonne überfordert.

Im Falle der kontinuierlichen Arbeitsweise, z. B. der des oben beschriebenen integrierten Systems, stellt sich das Problem einer zu hohen Konzentration des Methyljodids an irgendeiner Stelle nicht, da das Methyljodid entsprechend dem Wesen eines kontinuierlichen Verfahrens laufend abgeführt wird.

### Beispiel 1

Als Destillationskolonne diente eine mit Maschendrahtringen gefüllte Füllkörperkolonne von 30 mm lichter Weite und 80 cm Höhe. Die Trennstufenzahl dieser Kolonne, die kontinuierlich bei Normaldruck betrieben wurde, betrug 28 theoretische Böden.

Dem 8. Boden (von unten gezählt) wurden stündlich 60 g Acetaldehyd zugeführt, der mit rd. 300 ppm Methyljodid verunreinigt war. Die Temperatur an diesem Boden betrug etwa 15°C. Auf Höhe des 18. Bodens wurden stündlich 120 g Isopentan in die Kolonne eingeleitet.

Bei einem Rücklaufverhältnis von 5 wurde vom Kopf der Kolonne (11°C) stündlich ein Gemisch aus 49 g Acetaldehyd und 120 g Isopentan entnommen, welches nur noch etwa 0,5 ppm Methyljodid enthielt. Im Sumpf der Kolonne (21°C) fielen stündlich 11 g Acetaldehyd an, der mit etwa 0,16 Gew.-% Methyljodid verunreinigt war.

### Beispiel 2

Dieses Beispiel wurde in einer Versuchsapparatur gemäß der Figur unter Normaldruck ausgeführt. Sämtliche Kolonnen waren als Füllkörperkolonnen ausgebildet. Die Kolonne I war 2 m hoch, hatte einen inneren Durchmesser von 5 cm und 40 theoretische Böden. Die Kolonne II war 2,50 m hoch, hatte einen Innendurchmesser von 5 cm und 60 theoretische Böden und die Kolonne III war 0,8 m hoch, hatte einen Innendurchmesser von 5 cm und 12 theoretische Böden. Auf Höhe des 30. theoretischen Bodens (von unten gezählt), bei welchem die Temperatur 61°C betrug, wurden der Kolonne I stündlich 300 g eines Reaktionsgemisches zugeführt, welches der Homogolisierung von Methanol entstammte.

Die Homologisierung wurde bei 300 bar und 125°C in Gegenwart von 0,3 Gew.-% Cobalt als Cobaltacetat und etwa 1 Gew.-% Methyljodid, bezogen auf die Menge des Reaktorinhalts, mit einem Gemisch aus 50 Vol.-% Wasserstoff und 50 Vol.-% Kohlenmonoxid vorgenommen, wobei ein Methanolumsatz von etwa 26% eingehalten wurde. Das erhaltene Reaktionsgemisch wurde nach Entspannung mit Luft begast, wobei der Cobaltkomplex zerstört wurde. Anschließend wurde das Gemisch von dem entstandenen Cobaltsalz abdestilliert. Dieses Gemisch hatte folgende Zusammensetzung:

54 Gew.-% (162 g) Methanol
7 Gew.-% (21 g) Acetaldehyd
9 Gew.-% (27 g) Acetaldehyddimethylacetal
7 Gew.-% (21 g) Methylacetat
15 Gew.-% (45 g) Wasser
6 Gew.-% (18 g) Ethanol
2 Gew.-% (6 g) sonstige Produkte
rd. 30 ppm Methyljodid.

Auf Höhe des 35. Bodens (48°C) wurden stündlich 100 g 1gew.-%ige wäßrige Schwefelsäure, die noch etwas Ethanol und sonstige Produkte enthielt, in die Kolonne I eingeführt.

Bei einem Rücklaufverhältnis von 5 wurde stündlich ein Gemisch aus 34 g Acetaldehyd, 21 g Methylacetat, 0,5 g Acetaldehyddimethylacetal und 0,1 g Methanol am Kolonnenkopf (29°C) abgezogen, welches etwa 300 ppm Methyljodid enthielt.

Dieses Gemisch wurde auf Höhe des 50. Bodens (25—30°C) in die Kolonne II gegeben, und auf Höhe des 40. Bodens wurden stündlich 100 g Isopentan zugeführt.

Bei einem Rücklaufverhältnis von 7 wurde am Kopf der Kolonne II stündlich ein Gemisch aus 34 g Acetaldehyd und 99 g Isopentan, dessen Methyljodidgehalt unter 10 ppm lag, entnommen.

Als Sumpfprodukt (58°C) der Kolonne II fielen stündlich 18,5 g Methylacetat an, die neben etwas Methanol und Acetataldehyddimethylacetal nur noch 30 ppm Methyljodid enthielten.

Auf Höhe des 30. Bodens wurde der Kolonne II als dampfförmiger Seitenabzug stündlich ein Gemisch aus 2,5 g Methylacetat und 1 g Isopentan entnommen, das etwa 0,4 Gew.-% Methyljodid enthielt.

Auf Höhe des 15. Bodens (75°C) wurde der Kolonne I ein dampfförmiger Seitenabzug entnommen, der in der Kolonne III in 181 g Methanol als Kopfprodukt und 64 g Sumpfprodukt, das aus 40 g Wasser, 18 g Ethanol und 6 g sonstiger Produkte bestand, zerlegt wurde.

Dieses Sumpfprodukt wurde mit dem Sumpfprodukt der Kolonne I (110°C), welches aus 100 g der wäßrigen Schwefelsäure bestand, vereinigt, wonach 64 g der vereinigten Sumpfprodukte aus dem Kreislauf entfernt wurden. Anschließend wurde zur Konstanthaltung der Säurekonzentration wieder etwas Schwefelsäure in den Kreislauf gegeben.

### Patentansprüche

1. Verfahren zur Abtrennung organischer Jod-Verbindungen von Acetaldehyd, dadurch gekennzeichnet, daß man den Acetaldehyd einer Azeotropdestillation mit einem Kohlenwasserstoff, der unter Normaldruck bei 25—55°C siedet, unterwirft.

2. Verfahren zur Abtrennung organischer Jod-Verbindungen von Acetaldehyd im Rahmen der Gewinnung von Acetaldehyd aus Homologisierungsgemischen des Methanols, welche neben Acetaldehyd im wesentlichen Acetaldehyddimethylacetal, Methanol, Methylacetat und Wasser enthalten, wobei man

a) das Homologisierungsgemisch bei einer Temperatur in den mittleren Teil einer Kolonne I gibt, bei welcher das Methylacetat oder sein azeotropes Gemisch mit Methanol, nicht aber das Methanol gänzlich verdampft,

b₁) im Gegenstrom zu den aufsteigenden Dämpfen flüssige wäßrige Säure leitet, die im oberen Viertel der Kolonne I zugeführt wird und welche eine Spaltung des Acetaldehyddimethylacetals in Acetaldehyd und Methanol bewirkt, oder

b₂) anstelle der wäßrigen Säure lediglich Wasser verwendet und die Acetspaltung mittels eines festen sauren Ionenaustauschers bewirkt,

c) vom Kopf der Kolonne I ein dampfförmiges, Gemisch aus Methylacetat und Acetaldehyd abzieht, welches in einer Kolonne II in seine Komponenten zerlegt wird,

d) im unteren Viertel der Kolonne I ein flüssiges Gemisch aus Methanol und Wasser abzieht, welches in einer Kolonne III in seine Komponenten zerlegt wird, und wenn man

e) einen Teil der im Sumpf der Kolonne I anfallenden wäßrigen Säure (Variante b₁) bzw. des Wassers (Variante b₂), zusammen mit untergeordneten Mengen sonstiger Produkte aus dem System entfernt und den restlichen Teil dieser wäßrigen Säure bzw. des Wassers mit dem im Sumpf der Kolonne III anfallenden Wasser vereinigt und die vereinigte wäßrige Lösung nach Zugabe einer Säure wieder in das obere Viertel der Kolonne I zurückführt,

dadurch gekennzeichnet, daß man im Verfahrensschritt (c) die Trennung von Methylacetat und Acetaldehyd in Gegenwart eines Kohlenwasserstoffs, der unter Normaldruck bei 25—55° C siedet, vornimmt, das Acetaldehyd/Kohlenwasserstoff-Gemisch dem Kopf der Kolonne II, eine die Jodverbindung, Methylacetat und den Kohlenwasserstoff enthaltende Fraktion der Kolonne II als Seitenabzug und die Methylacetat-Fraktion der Kolonne II als Sumpfprodukt entnimmt.

3. Verfahren nach den Ansprüchen 1 oder 2, dadurch gekennzeichnet, daß man als Kohlenwasserstoff Methylbutan verwendet.

**Claims**

1. A process for removing organic iodine compounds from acetaldehyde, wherein the acetaldehyde is subjected to azeotropic distillation with a hydrocarbon which has a boiling point, under atmospheric pressure, of from 25 to 55° C.

2. A process for removing organic iodine compounds from acetaldehyde for the purpose of obtaining acetaldehyde from homologization mixtures of methanol which, in addition to acetaldehyde, essentially contain acetaldehyde dimethyl acetal, methanol, methyl acetate and water, in which

a) the homologization mixture is introduced into the middle section of a column I at a temperature at which the methyl acetate or its azeotropic mixture with methanol, but not the methanol, vaporizes completely,

b₁) liquid aqueous acid is fed, in counter-current to the rising vapors, into the top quarter of column I and splits the acetaldehyde dimethyl acetal into acetaldehyde and methanol, or

b₂) instead of the aqueous acid, only water is used and the acetal is split by means of a solid acidic ion exchanger,

c) a vaporous mixture of methyl acetate and acetaldehyde is taken off at the top of column I and is separated into its components in a column II,

d) a liquid mixture of methanol and water is taken off from the bottom quarter of column I and is separated into its components in a column III, and

e) some of the aqueous acid (embodiment b₁) obtained in the bottom of column I or some of the water (embodiment b₂) is removed from the system with minor amounts of other products, and the remainder of this aqueous acid or of the water is combined with the water obtained in the bottom of column III, and the aqueous solution is recycled to the top quarter of column I, after addition of an acid, wherein the separation of methyl acetate and acetaldehyde in process step (c) is carried out in the presence of a hydrocarbon with a boiling point, under atmospheric pressure, of from 25 to 55° C, and the acetaldehyde/hydrocarbon mixture is taken off from the top of column II, a fraction containing the iodine compound, methyl acetate and the hydrocarbon is taken off as a side-stream from column II, and the methyl acetate fraction is taken off as the bottom product from column II.

3. A process as claimed in claim 1 or 2, wherein methylbutane is used as the hydrocarbon.

**Revendications**

1. Procédé pour la séparation de composés organiques iodés de l'aldéhyde acétique, caractérisé en ce que l'aldéhyde acétique est soumis à une distillation azéotrope avec un hydrocarbure qui, à la pression normale, bout entre 25 et 55° C.

2. Procédé pour la séparation de composés organiques iodés de l'aldéhyde acétique dans le cadre de l'extraction de l'aldéhyde acétique de mélanges d'homologisation du méthanol, contenant essentiellement, à côté de l'aldéhyde acétique, du diméthyl-acétal de l'aldéhyde acétique, du méthanol, de l'acétate de méthyle et de l'eau, dans lequel:

a) on introduit le mélange d'homologisation dans la partie médiane d'une colonne I à une température, à laquelle est vaporisé l'acétate de méthyle ou le mélange azéotrope de celui-ci et du méthanol, mais non la totalité du méthanol;

b$_1$) on introduit dans le quart supérieur de la colonne I un acide aqueux liquide, qui s'écoule en contre-courant des vapeurs ascendantes et provoque la décomposition du diméthyl-acétal de l'aldéhyde acétique en aldéhyde acétique et méthanol, ou

b$_2$) on remplace l'acide aqueux par de l'eau seule et on provoque la décomposition de l'acétal à l'aide d'un échangeur d'ions acide solide;

c) on soutire en tête de la colonne I un mélange gazeux d'acétate de méthyle et d'aldéhyde acétique, qui est séparé dans une colonne II en ses composants;

d) on soutire du quart inférieur de la colonne I un mélange liquide de méthanol et d'eau, qui est décomposé en ses composants dans une colonne III;

e) une partie de l'acide aqueux (variante b$_1$) ou de l'eau (variante b$_2$) recueilli dans la partie inférieure de la colonne I étant éliminée du système avec des quantités mineures d'autres produits, la fraction restante de l'acide aqueux ou de l'eau étant ajoutée à l'eau recueillie dans la partie inférieure de la colonne III et cette solution aqueuse étant recyclée, après addition d'un acide, dans le quart supérieur de la colonne I,

caractérisé en ce que la séparation de l'acétate de méthyle de l'aldéhyde acétique, dans la phase opératoire (c), est réalisée en présence d'un hydrocarbure qui, à la pression normale, bout entre 25 et 55°C, le mélange aldéhyde acétique-hydrocarbure étant soutiré au sommet de la colonne II, une fraction contenant les composés iodés, de l'acétate de méthyle et de l'hydrocarbure étant soutirée latéralement de la colonne II et la fraction acétate de méthyle obtenue comme produit résiduel dans la partie inférieure de la colonne II.

3. Procédé suivant la revendication 1 ou 2, caractérisé en ce que l'hydrocarbure mis en œuvre est le méthyl-butane.

Me = Methanol
Ald = Acetaldehyd
Ald-Me = Acetaldehyddimethylacetal
Me-Ac = Methylacetat
J = Methyljodid
IP = Isopentan
s = sonstige
( ) = geringe Mengen